# FASCICULE DE BREVET EUROPEEN

(11) **EP 0 935 448 B1**
(45) Date de publication et mention de la délivrance du brevet: **03.07.2002**
(21) Numéro de dépôt: 97944952.7
(22) Date de dépôt: 10.10.1997
(51) Int. Cl.: A61F 2/16

(54) **IMPLANT INTRAOCULAIRE A OPTIQUE SOUPLE ET A ANSE UNIQUE CIRCULAIRE RIGIDE**
INTRAOKULARES LINSENIMPLANTAT MIT EINEM WEICHEN OPTISCHEN TEIL UND EINEM EINSTÜCKIGEN KREISFÖRMIGEN STEIFEN BÜGEL
INTRAOCULAR IMPLANT WITH FLEXIBLE OPTICAL PART AND SINGLE CIRCULAR LOOP

(30) Priorité: 10.10.1996 FR 9612360
(43) Date de publication de la demande: 18.08.1999
(73) Titulaire: CORNEAL LABORATOIRES, 75012 Paris (FR)
(72) Inventeur: BAIKOFF, Georges, F-13007 Marseille (FR); ORTUNO, Angel, F-74330 Choisy (FR)
(74) Mandataire: Dronne, Guy
(86) Numéro de dépôt international: FR9701807
(87) Numéro de publication internationale: WO9815239

(56) Documents cités:
- EP-A- 0 338 847
- EP-A- 0 438 043
- WO-A-94/28825
- US-A- 4 494 254
- US-A- 4 950 290

## Description

La présente invention concerne un implant intraoculaire pour correction de l'aphakie comportant une partie optique sensiblement circulaire réalisée en un premier matériau souple permettant son pliage selon un diamètre et une partie haptique.

Les implants intraoculaires constituent un système optique correcteur de la vision de l'oeil humain qui peut dans un certains nombre de cas se substituer à des lentilles cornéennes ou à des verres correcteurs externes. Un implant intraoculaire se compose essentiellement d'une partie optique de forme générale circulaire ou légèrement ovalisée qui constitue le système optique correcteur proprement dit, et par une partie haptique qui sert à la mise en place, à la fixation et au maintien en position correcte de la partie optique à l'intérieur de l'oeil.

De nouvelles techniques opératoires permettent de diminuer l'incision pratiquée au niveau de l'oeil. Ainsi, dans le cas de l'opération de la cataracte, la technique opératoire dite de phaco-émulsification permet l'ablation du cristallin opaque par l'introduction dans l'oeil d'une sonde à ultrasons présentant un système d'irrigation/aspiration. Par l'action combinée des ultrasons et du flux de solution saline équilibrée, on vient retirer le cristallin par émulsification.

Cette technique opératoire présente l'avantage, notamment par rapport aux techniques antérieures, de ne nécessiter que la réalisation d'une incision de dimension réduite dans la cornée grâce à un couteau précalibré à 2 à 4 mm pour introduire dans l'oeil les instruments nécessaires à cette ablation. On comprend dès lors qu'il est intéressant de disposer d'implants qui puissent être introduits dans l'oeil par l'incision effectuée pour l'opération de phaco-émulsification, c'est-à-dire par une incision dont la longueur est de l'ordre de 3 à 4 mm.

C'est pourquoi de nouveaux implants intraoculaires dits souples ont été développés. Ils présentent une partie optique réalisée dans un matériau souple qui permet de plier la partie optique avant l'introduction de l'implant dans l'oeil par l'incision, la partie optique reprenant sa forme initiale après sa mise en place dans l'oeil. Actuellement deux grands types de produits sont utilisés pour réaliser les optiques souples. Ces produits sont habituellement désignés sous le nom générique, d'une part, d'acryliques souples tels que le pHEMA et d'autre part, de gel de polysiloxane. Ces matériaux présentent les propriétés optiques requises et sont par ailleurs biocompatibles.

L'ablation du cristallin conduit à la déformation par contraction du sac capsulaire et par conséquent à son opacification. La déformation du sac capsulaire peut conduire à la disparition de la symétrie circulaire du sac capsulaire et donc à la perturbation de la vision de l'oeil par altération des axes de symétrie du système optique constitué par le sac capsulaire et l'implant intraoculaire.

La partie haptique des implants intra-oculaires participe au maintien de la forme initiale du sac capsulaire, en particulier sa périphérie circulaire.

Le phénomène de déformation du sac capsulaire est non négligeable dans le cas où la partie haptique est constituée par deux anses identiques diamétralement opposées et symétriques l'une de l'autre par rapport au centre optique car dans ce cas les zones de contact entre les anses et la paroi interne du sac capsulaire sont deux zones limitées qui doivent supporter entièrement la pression exercée par les deux anses.

De plus il est à noter que la déformation par contraction du sac capsulaire entraîne un risque de déchirement partiel du ligament zonulaire qui retient le sac capsulaire.

C'est pourquoi on a cherché à développer des implants avec une haptique circulaire qui sera en contact avec la paroi intérieure du sac capsulaire sur des zones relativement étendues.

Le document **D1=: EP-A-0338847** décrit in implant intraoculaire comportant une partie optique sensiblement circulaire et une partie haptique comprenant un corps réalisé en un différent matériau et sensiblement annulaire, sensiblement concentrique et coplanaire à la partie optique, ledit corps au moins présentant une ouverture, une première extrémité libre et une deuxième extrémité de raccordement.

Une première solution consiste à utiliser une partie haptique constituée par deux anses rigides identiques symétriques l'une de l'autre par rapport à un diamètre de la partie optique. Cette configuration impose des anses avec un petit diamètre pour éviter qu'elles se déforment trop et empêcher un décentrement de l'implant dans le sac capsulaire. Des anses de grande longueur sont également nécessaires pour essayer d'obtenir une zone de contact la plus large possible. Dans ce cas, le problème de la contraction du sac capsulaire entraîne le risque d'un croisement c'est-à-dire d'un chevauchement entre les extrémités libres des anses, ce qui pourrait conduire à la perforation du sac.

Une deuxième solution consiste à utiliser une anse annulaire unique rattachée à la périphérie de la partie optique par un bras de raccordement. Dans les implants réalisés selon cette configuration, soit la partie haptique est rigide et on ne peut plus obtenir une largeur d'implant plié minimale, ce qui était réalisable avec deux anses rigides symétriques, soit la partie haptique est souple et pliable mais le maintien de l'implant dans l'oeil par cette partie haptique souple est insuffisant pour que l'implant intraoculaire reste centré dans le sac capsulaire sans s'affaisser.

Un objet de la présente invention est de réaliser un implant intraoculaire qui permette à la fois un pliage selon un diamètre de la partie optique de façon à obtenir une largeur d'implant plié de l'ordre de 3 ou 4 mm et un maintien de l'implant dans l'oeil par la partie haptique qui évite la déformation du sac capsulaire et le décentrement ou déplacement de la partie optique de l'implant.

Pour atteindre ce but, l'implant intraoculaire selon l'invention comporte une partie optique sensiblement circulaire réalisée en un premier matériau souple permettant son pliage selon un diamètre et une partie haptique rigide, cet implant se caractérisant en ce que la partie haptique comprend un corps sensiblement annulaire, sensiblement concentrique et coplanaire à la partie optique, ledit corps présentant une ouverture, une première extrémité libre et une deuxième extrémité de raccordement, ledit corps étant réalisé en un deuxième matériau plus rigide que le premier matériau, et une base reliant de façon sensiblement radiale la deuxième extrémité du corps de la partie haptique à une zone périphérique de la partie optique, ladite base étant raccordée à la périphérie de la partie optique sur une longueur suffisante correspondant à un angle b inférieur à 180 degrés pour éviter un mouvement de torsion de la partie optique par rapport à la partie haptique.

On comprend qu'ainsi, la partie optique qui est souple peut être pliée selon un diamètre, ce qui permet effectivement l'introduction de l'implant dans l'oeil par une incision de petite dimension. En revanche, la partie haptique étant sensiblement circulaire et rigide, on a un bon maintien du sac capsulaire. Enfin, grâce à la structure de la base de la partie haptique, on évite un déplacement de la partie optique par rapport à la partie haptique.

Pour introduire l'implant, on fait tout d'abord pénétrer la partie optique pliée dans l'incision puis ensuite la base de la partie haptique et enfin on fait rentrer le corps annulaire par rotation de l'implant autour d'un axe parallèle à son axe optique tout en maintenant, de préférence, le corps annulaire sensiblement coplanaire à la partie optique.

Selon un mode préféré de réalisation, l'implant intraoculaire se caractérise en ce que la partie haptique comprend un corps sensiblement annulaire, sensiblement concentrique et coplanaire à la partie optique, ledit corps au moins présentant une ouverture, une première extrémité libre et une deuxième extrémité de raccordement, ledit corps étant réalisé en un deuxième matériau plus rigide que le premier matériau, et une base reliant de façon sensiblement radiale la deuxième extrémité du corps de la partie haptique à une zone périphérique de la partie optique, ladite base étant réalisée avec le premier matériau et présentant une zone latérale comprenant une armature rigide (30a) réalisée avec le deuxième matériau qui prolonge ledit corps au moins jusqu'à la périphérie de la partie optique.

Selon une caractéristique essentielle de ce mode de réalisation de l'invention, pour permettre le pliage de l'implant avec l'obtention d'une largeur d'implant plié minimale tout en permettant un maintien centré de l'implant dans l'oeil, il est prévu que ladite base présente une première zone latérale comprenant une première armature rigide réalisée avec le deuxième matériau qui prolonge ledit corps au moins jusqu'à la périphérie de la partie optique et une deuxième zone latérale comprenant une deuxième armature rigide réalisée avec le deuxième matériau, ne s'étendant pas sur toute la longueur de cette deuxième zone latérale et adjacente à la partie optique de sorte qu'il existe au moins un diamètre de la partie optique qui se prolonge dans la base de la partie haptique sans couper lesdites armatures .

On comprend que la partie haptique de l'implant selon l'invention comporte un corps suffisamment rigide annulaire d'une seule pièce permettant un maintien efficace de l'implant dans l'oeil par une déformation minimale de ce corps, et une répartition maximale de la pression exercée par la partie haptique sur la paroi du sac capsulaire.

On comprend également que, selon le mode préféré de réalisation, la structure bimatériau de la base de la partie haptique de l'implant permet, d'une part, de plier l'implant selon un diamètre de la partie optique traversant cette base de façon à obtenir une largeur d'implant plié minimale et, d'autre part, un décentrement minimum grâce aux armatures rigides des zones latérales de la base de la partie haptique qui empêchent une déformation trop importante de celle-ci.

Grâce à la première armature rigide radiale de la base de la partie haptique qui prolonge le corps annulaire jusqu'à la périphérie de la partie optique, la partie optique est maintenue centrée autour de l'axe optique de l'oeil. La deuxième armature rigide et la partie centrale souple de la base de la partie haptique augmentent, d'une part, la résistance mécanique en flexion de la première armature reliant la partie optique au corps annulaire et, d'autre part, le moment d'inertie de la base de la partie haptique. Cette augmentation du moment d'inertie évite tout pivotement autour du diamètre de la partie optique qui se prolonge dans la partie centrale souple de la base de la partie haptique. En effet un tel pivotement désaxerait l'axe optique de l'implant par rapport à l'axe optique de l'oeil du fait qu'ils ne seraient plus parallèle entre eux et cela endommagerait fortement la correction optique apportée par l'implant.

Il est à noter que l'implant selon l'invention n'est pas forcément destiné à être placé uniquement dans le sac capsulaire, il peut aussi, par exemple, être positionné dans la chambre postérieure de l'oeil, en s'appuyant dans le sillon irrido ciliaire (sulcus).

L'invention sera mieux comprise et les caractéristiques secondaires et leurs avantages apparaîtront au cours de la description de modes préférés de réalisations donnés ci-dessous à titre d'exemple.

Il est entendu que la description et les dessins ne sont données qu'à titre descriptif et non limitatif.

Il sera fait référence aux dessins annexés dans lesquels :
- la figure 1 est une vue de face d'un implant intra-oculaire selon un mode préféré de réalisation de l'invention présentant une partie haptique annulaire bimatériau ;
- la figure 2 est une vue de côté de l'implant intra-oculaire représenté sur la figure 1 ;
- la figure 3 est une coupe transversale suivant la direction III-III de la figure 1 et représente, à l'échelle agrandie, la répartition des différents matériaux dans la section de la base de la partie haptique ;
- la figure 4 est une vue identique à celle de la figure 3 correspondant à une variante de réalisation;
- La figure 5 présente une première variante de réalisation de l'implant de la figure 1;
- La figure 6 illustre un deuxième mode de réalisation de l'invention; et
- La figure 7 représente une deuxième variante de réalisation de l'implant de la figure 1.

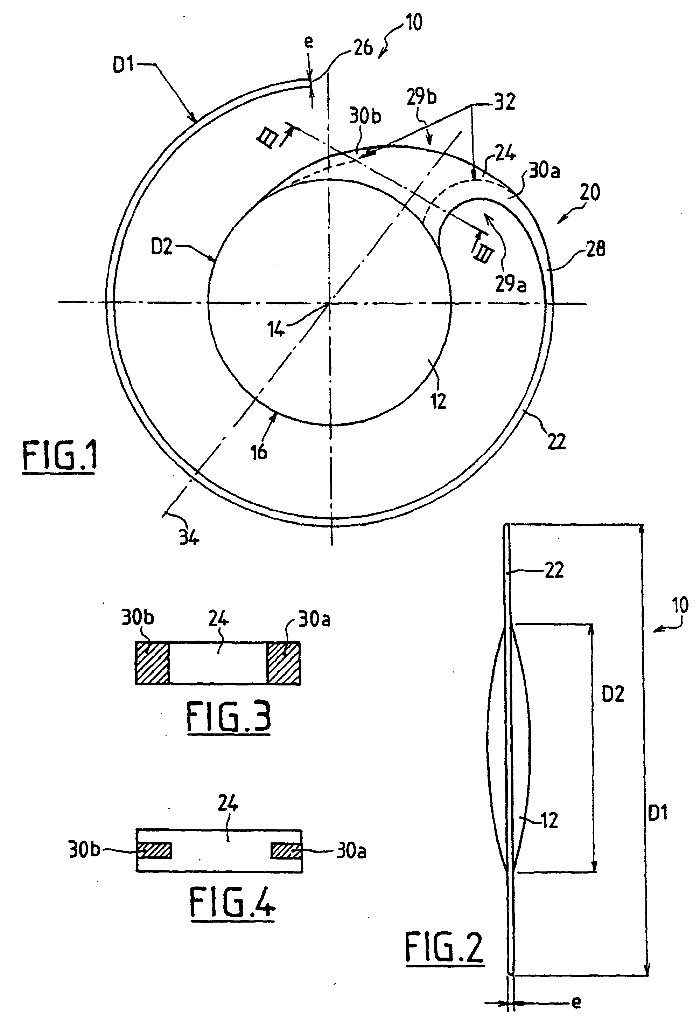

Selon un mode préféré de réalisation, représenté sur la figure 1, l'implant intra-oculaire 10 comporte une partie optique 12 sous la forme d'une lentille qui peut être biconvexe présentant un centre optique 14 et une périphérie bombée 16. On peut décomposer la partie haptique 20 de l'implant intra-oculaire en un corps 22 annulaire et une base 24 reliant la partie optique 12 au corps 22 de la partie haptique 20.

La partie optique 12 de l'implant oculaire est réalisée en un premier matériau souple permettant le pliage de cette partie optique, par exemple un copolymère à base de pHEMA ou un matériau comprenant un acrylate hydrophile ou du gel de silicone. Le corps 22 de la partie haptique 20 forme un anneau ouvert réalisé en un deuxième matériau qui est rigide, tel que du PMMA. Le corps annulaire 22 de la partie haptique 20 s'étend depuis une extrémité libre 26 jusqu'à une extrémité de raccordement 28 qui va être reliée à la partie optique 12, l'arc de cercle compris entre ces deux extrémités formant, de préférence, un angle au centre au moins sensiblement égal à 270°. Il est prévu que l'épaisseur e du corps annulaire 22 soit sensiblement constante, de préférence de l'ordre de 0,20 mm, le diamètre D1 de ce corps annulaire 22 étant de préférence de l'ordre de 10 à 11 mm.

Classiquement, le diamètre D2 de la partie optique 12 est de l'ordre de 5 à 6 mm.

La base 24 de la partie haptique 20 va maintenant être décrite en relation avec la figure 1. Cette base 24 permet de raccorder de façon sensiblement radiale la partie optique 12 au corps annulaire 22 de la partie haptique 20. Cette base 24 est essentiellement constituée du matériau souple précédemment cité et possède, dans ses deux zones latérales 29a et 29b s'étendant depuis la périphérie 16 de la partie optique en direction du corps annulaire 22 de la partie haptique 20, deux armatures rigides 30a et 30b constituées du second matériau, les limites entre le premier et le deuxième matériaux étant représentées par les lignes 32 en traits pointillés de la figure 1.

On peut concevoir une seule armature rigide radiale 30a prolongeant de façon radiale le corps annulaire 22 jusqu'à la périphérie 16 de la partie optique.

La première armature rigide 30a prolonge la partie optique 12 au corps annulaire 22 en s'étendant de façon continue et radiale depuis l'extrémité de la première zone latérale 29a de la base 24 de la partie haptique qui est adjacente à la périphérie 16 de la partie optique, du côté le plus proche de l'extrémité de raccordement 28 du corps annulaire 22, jusqu'à cette extrémité de raccordement 28. La deuxième armature rigide 30b s'étend sur la deuxième zone latérale 29b de la base 24 de la partie haptique 20 depuis la périphérie 16 de la partie optique 12 jusqu'à environ le milieu de cette zone latérale, sans effectuer de jonction avec le deuxième matériau que ce soit avec la première armature 30a ou avec l'extrémité de raccordement 28 du corps annulaire 22.

De cette façon, le bord de la base 24 de la partie haptique 20 n'est pas entièrement constituée du deuxième matériau rigide et il existe au moins un diamètre de la partie optique 12 qui se prolonge dans la base 24 de la partie haptique 20 sans croiser le deuxième matériau du fait qu'il ne rencontre que le premier matériau.

On pourra donc plier l'implant selon un axe de pliage 34 passant par ledit diamètre de la partie optique et la base de la partie haptique sans couper une des armatures rigides.

La base 24 possède une direction générale radiale de façon à raccorder la partie optique au corps annulaire 22 de la partie haptique.

Les armatures rigides 30a et 30b possèdent, de préférence, une épaisseur et une largeur minimale de 0,1 mm, la zone de la base 24 de la partie haptique réalisant la jonction avec la partie optique 12 possède une largeur pouvant varier entre 1 mm et le diamètre D2 de la partie optique et une épaisseur entre 0,15 et 0,4 mm.

De préférence, le contour externe des zones latérales de la base 24 de la partie haptique 20 est courbe. Le rayon de courbure du contour extérieur de la première zone latérale 29a est choisi et positionné de façon à former, de préférence, un congé au niveau de la zone de jonction entre la zone périphérique 16 de la partie optique et la base 24 de la partie haptique. Le rayon de courbure du contour extérieur de la deuxième zone latérale 29b est choisi et positionné de façon que ce contour prolonge sensiblement tangentiellement le contour externe de la partie optique 12. Le raccordement entre la base 24 de la partie haptique 20 et l'extrémité de raccordement 28 du corps annulaire 22 s'effectue également de préférence de façon sensiblement tangentielle.

Cette forme arrondie de la base 24 de la partie haptique permet une meilleure répartition des contraintes sur cette zone d'où une déformation moins localisée et un risque de décentrement de l'implant dans l'oeil qui est diminué.

On privilégiera des armatures rigides 30a et 30b possédant une largeur sensiblement constante dont le profil suit la forme du contour externe des zones latérales de la base 24 de la partie haptique 20.

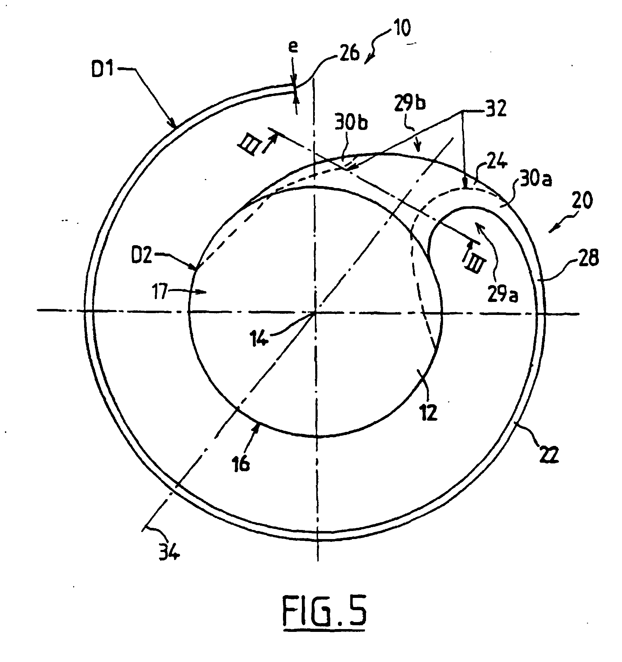

On va maintenant décrire, en relation avec les figures 3 et 4, la répartition du premier et du second matériau, respectivement souple et rigide, dans la section transversale de la base 24 de la partie haptique au niveau d'une zone comprenant les deux armatures 30a et 30b. Dans les deux cas, on trouve que le deuxième matériau rigide constituant les armatures 30a et 30b débouche à l'extérieur de la base 24 de la partie haptique 20 en direction des faces latérales de cette base 24.

Dans le cas de la figure 3, le deuxième matériau rigide remplit toute l'épaisseur de la base 24 mais il est possible de prévoir, comme dans le cas de la figure 4, que ce deuxième matériau ne remplisse qu'une partie de l'épaisseur des parties des zones latérales 29a et 29b de cette base 24 comprenant les armatures 30a et 30b, de préférence une zone médiane de l'épaisseur entourée de part et d'autre par le premier matériau souple qui constitue la partie essentielle de la base 24 de la partie haptique 20.

La périphérie 16 de la partie optique 12 est raccordée à la base 24 de la partie haptique 20 selon un arc de cercle d'angle au centre au moins sensiblement égal à 90°.

La variante de réalisation représentée sur la figure 5 consiste à prolonger les armatures 30a et 30b dans la zone périphérique 17 de la partie optique 12 sur une longueur de la périphérie pouvant aller jusqu'à environ 3 mm.

On va maintenant décrire un procédé de fabrication permettant la réalisation de l'implant intra-oculaire 10 selon l'invention. On utilise une plaque réalisée dans le deuxième matériau rigide dont les dimensions sont suffisantes pour inclure tout l'implant et l'on crée, par usinage de cette plaque, un évidement correspondant à la partie optique 12 et à la zone de la base 24 de la partie haptique 20 qui seront uniquement constituées par le premier matériau souple. Ensuite, on moule la partie optique 12 et la base 24 de la partie haptique dans un dispositif de moulage conformé selon la géométrie recherchée. Pour cette étape de moulage, on pourra se reporter, par exemple, aux demandes de brevet français n° 94 12274 et 94 12275 correspondant à la demande PCT n° WO-A-96/11792. Au cours de cette étape de moulage à chaud, on va réaliser une interpénétration des réseaux moléculaires des premier et deuxième matériaux macromoléculaires de façon à obtenir une liaison entre matériaux souple et rigide qui soit très forte. On obtient ainsi une solidarisation entre le deuxième matériau constituant les armatures rigides et le premier matériau qui se trouve, d'une part, dans la zone de la périphérie de la partie optique adjacente à ces armatures et, d'autre part, dans les zones latérales de la base de la partie haptique.

Pour finir la réalisation de cet implant intra-oculaire, on va usiner le premier matériau, si cela est possible et necessaire, de façon à obtenir les caractéristiques optiques de la partie optique 12 qui soient conformes à celles de l'implant que l'on souhaite réaliser et on réalise l'usinage précis du deuxième matériau c'est-à-dire de la base 24 et du corps annulaire 22 de la partie haptique 20.

D'éventuels traitements ultérieurs peuvent être réalisés sur cet implant oculaire conformément aux traitements habituellement réalisés sur ce type d'implant à optique souple.

De même, des traitements convenables peuvent être réalisés sur la partie haptique de l'implant pour lui conférer des propriétés spécifiques.

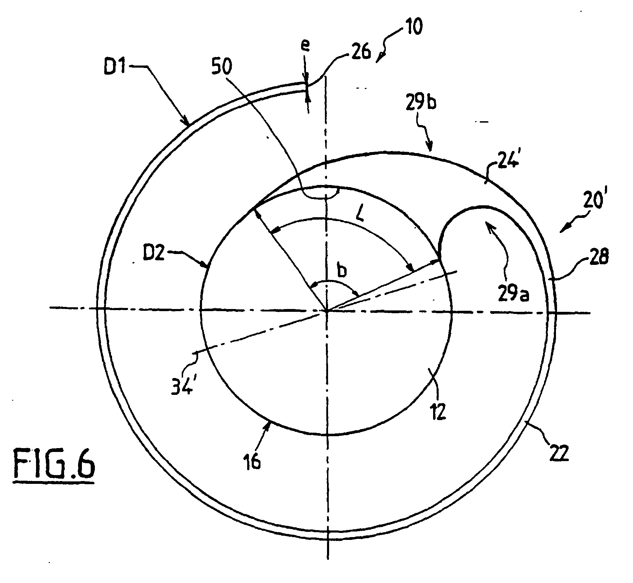

En se référant maintenant à la figure 6, on va décrire un deuxième mode de réalisation de l'implant. Celui-ci comprend toujours une optique circulaire 12 réalisée en un matériau souple et une partie haptique 20' constituée par un corps 22 annulaire et une base 24' reliant la partie optique au corps 22.

Selon ce mode de réalisation, le corps annulaire 22 est identique au corps 22 des figures 1 et 5, seule la base 24' étant modifiée.

Plus précisément, la base 24' est entièrement réalisée avec le même matériau que le corps annulaire 22. Il peut être du PMMA.

La base est raccordée à la périphérie 16 de la partie optique par tout moyen convenable compatible avec les matériaux constitutifs de l'optique et de l'haptique. De préférence, mais non exclusivement, la liaison pourra être obtenue par interpénétration de réseaux entre les deux matériaux.

Afin d'assurer une liaison mécanique suffisante entre les deux parties de l'implant, la longueur L de la zone de contact 50 doit être suffisante. L'angle au centre b correspondant à cette liaison est de préférence supérieur à 45 degrés. On comprend cependant que cet angle b doit être strictement inférieur à 180 degrés, pour laisser "libre" un diamètre de pliage de la partie optique 12. Sur la figure 6, on a représenté un diamètre possible de pliage 34'. D'autres diamètres de pliage pourraient bien sûr être utilisés à condition qu'il ne "coupe" pas la zone de contact 50.

La longueur L de la liaison entre la périphérie de la partie optique et la naissance de la base 24' de la partie optique permet également d'éviter que ne puissent se développer un couple de torsion au niveau de la base dont il pourrait résulter que le plan de la partie optique et le plan de la partie haptique ne soient plus sensiblement confondus. En outre, le caractère relativement massif et donc rigide de la base 24' de la partie haptique assure le centrage correct de l'optique par rapport au corps annulaire 22 de la partie haptique.

Des variantes du deuxième mode de réalisation peuvent être envisagées avec une base réalisée entièrement avec le même matériau souple ou premier matériau que la partie optique 22 ou bien une base comportant certaines zones réalisées avec le premier matériau, les autres zones étant réalisées avec le deuxième matériau constituant le corps annulaire. Selon la répartition entre matériau souple et matériau rigide, on peut envisager que l'axe de pliage traverse la base si cet axe ne rencontre pas le matériau rigide.

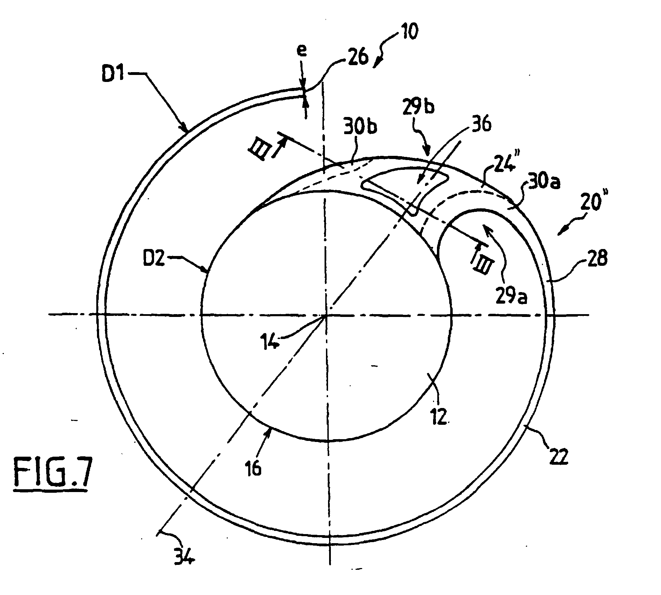

On va décrire maintenant la figure 7 qui illustre une deuxième variante du premier mode de réalisation (figure 1). L'implant de la figure 7 est identique à celui de la figure 1 sauf en ce qui concerne la base. On retrouve une partie optique 12 souple et une partie haptique 20" constituée d'un corps annulaire 22 rigide et d'une base 24" reliant la partie optique 12 au corps annulaire 22.

La base 24" comporte les deux armatures rigides 30a et 30b de part et d'autre de la zone centrale réalisée dans le premier matériau,celle-ci possédant un évidement 36. Cet évidement 36 est réalisé à l'écart des armatures rigides et n'entraîne pas de fragilisation sensible de la base 24" de sorte que la partie optique 12 et le corps annulaire 22 restent sensiblement coplanaires.

Grâce à la présence de cet évidement, lorsque l'implant a été mis en place dans l'oeil, les portions des parois antérieure et postérieure du sac capsulaire qui se trouvent face à l'évidement, de part et d'autre de la base 24", vont pouvoir adhérer l'une à l'autre et fusionner ensemble. Ainsi, la stabilité de la position de l'implant intraoculaire dans le sac capsulaire est renforcée, tout décentrement ou rotation de l'implant par rapport au sac capsulaire est empêché de sorte que l'axe optique de la partie optique 12 reste aligné avec l'axe optique du globe oculaire. De plus, puisque cet évidement 36 sert de point d'accrochage entre l'implant et le sac capsulaire, les forces de rappel exercées par le corps annulaire 22 sur le sac capsulaire pourront être moins importantes.

La variante de réalisation consistant à prévoir un évidement 36 dans la zone centrale de la base peut être réalisée dans tous les autres cas de figure décrits précédemment, c'est-à-dire avec une base 24 possédant des armatures (figures 1 et 5), une base 24' sans armatures (figure 6) et quelle que soit la forme ou quel que soit le matériau constituant cette base.

Une autre caractéristique importante de la présente invention réside dans le fait que la base présente une largeur qui diminue régulièrement depuis la périphérie de la partie optique (largeur L) jusqu'à atteindre l'extrémité de raccordement 28 du corps annulaire 22 (largeur e).

## Revendications

1. Implant intraoculaire (10) comportant une partie optique (12) sensiblement circulaire réalisée en un premier matériau souple permettant son pliage selon un diamètre et une partie haptique (20; 20'; 20"), la partie haptique (20; 20'; 20") comprenant un corps sensiblement annulaire (22), sensiblement concentrique et coplanaire à la partie optique, ledit corps (22) au moins présentant une ouverture, une première extrémité libre (26) et une deuxième extrémité de raccordement (28), ledit corps (22) étant réalisé en un deuxième matériau plus rigide que le premier matériau, et une base (24; 24'; 24") reliant de façon sensiblement radiale la deuxième extrémité (28) du corps de la partie haptique à une zone périphérique (16) de la partie optique, ladite base (24; 24'; 24") étant raccordée à la périphérie de la partie optique sur une longueur suffisante correspondant à un angle au centre b inférieur à 180 degrés et supérieur à 45 degrés pour éviter un mouvement de torsion de la partie optique par rapport à la partie haptique (20; 20'; 20").

2. Implant intraoculaire selon la revendication 1, **caractérisé en ce que** la partie haptique (20) comprend un corps sensiblement annulaire (22), sensiblement concentrique et coplanaire à la partie optique, ledit corps (22) au moins présentant une ouverture, une première extrémité libre (26) et une deuxième extrémité de raccordement (28), ledit corps (22) étant réalisé en un deuxième matériau plus rigide que le premier matériau, et une base (24) reliant de façon sensiblement radiale la deuxième extrémité (28) du corps de la partie haptique à une zone périphérique (16) de la partie optique, ladite base (24) étant réalisée avec le premier matériau et présentant une zone latérale (29a) comprenant une armature rigide (30a) réalisée avec le deuxième matériau qui prolonge ledit corps au moins jusqu'à la périphérie (16) de la partie optique.

3. Implant intraoculaire selon la revendication 2, **caractérisé en ce que** ladite base (24) présente une première zone latérale (29a) comprenant une première armature rigide (30a) réalisée avec le deuxième matériau qui prolonge ledit corps au moins jusqu'à la périphérie (16) de la partie optique et une deuxième zone latérale (29b) comprenant une deuxième armature rigide (30b) réalisée avec le deuxième matériau, ne s'étendant pas sur toute la longueur de cette deuxième zone latérale et adjacente à la partie optique de sorte qu'il existe au moins un diamètre (34) de la partie optique qui se prolonge dans la base de la partie haptique sans couper lesdites armatures (30a, 30b).

4. Implant intraoculaire selon la revendication 3, **caractérisé en ce que** le contour externe desdites zones latérales (29a, 29b) de la base (24) de la partie haptique est courbe.

5. Implant intraoculaire selon l'une quelconque des revendications 3 ou 4, **caractérisé en ce que** le contour externe desdites zones latérales (29a, 29b) de la base (24) de la partie haptique est conformé de façon à former, au niveau de la zone de jonction entre la zone périphérique (16) de la partie optique et la base (24) de la partie haptique, un congé pour la première zone latérale (29a) et un prolongement tangentiel du contour externe de la partie optique (12) pour la deuxième zone latérale (29b).

6. Implant intraoculaire selon l'une quelconque des revendications 3 à 5, **caractérisé en ce que** les armatures rigides (30a, 30b) possèdent une largeur sensiblement constante qui suit la forme du contour externe des zone latérales (29a, 29b).

7. Implant intraolculaire selon l'une quelconque des revendications 3 à 6, **caractérisé en ce que** les armatures rigides (30a, 30b) débouchent sur les faces latérales de la base (24).

8. Implant intraoculaire selon l'une quelconque des revendications 3 à 7, **caractérisé en ce que** le deuxième matériau remplit au moins une partie de l'épaisseur des parties des zones latérales (29a, 29b) comprenant les armatures (30a, 30b).

9. Implant intraoculaire selon l'une quelconque des revendications précédentes, **caractérisé en ce que** le corps annulaire (22) couvre un arc de cercle d'angle au centre au moins sensiblement égal à 270°.

10. Implant intraoculaire selon l'une quelconque des revendications 2 à 9, **caractérisé en ce que** le ou les armatures rigides (30a, 30b) s'étendent jusque dans la zone périphérique (17) de la partie optique (12).

11. Implant intraoculaire selon l'une quelconque des revendications précédentes, **caractérisé en ce que** le deuxième matériau est du PMMA.

12. Implant intraoculaire selon l'une quelconque des revendications précédentes, **caractérisé en ce que** le premier matériau comprend un acrylate hydrophile.

13. Implant intraloculaire selon l'une quelconque des revendications précédentes, **caractérisé en ce que** le premier matériau est du gel de silicone.

14. Implant intraoculaire selon l'une quelconque des revendications précédentes, **caractérisé en ce que** la base (24") comporte un évidement (36) apte à permettre la fusion d'une portion des parois antérieure et postérieure d'un sac capsulaire d'un oeil dans lequel ledit implant est destiné à être logé.

## Patentansprüche

1. Intraokulares Implantat (10), das aus einem deutlich kreisrunden optischen Teil besteht (12), hergestellt aus einem weichen ersten Material, das sein Zusammenklappen entsprechend einem Durchmesser erlaubt, und einem greifbaren Teil (20; 20'; 20"). Der greifbare Teil (20; 20'; 20") besteht aus einem deutlich ringförmigen Korpus (22), deutlich konzentrisch und komplanar zum optischen Teil, der genannte Korpus (22) verfügt mindestens über eine Öffnung, ein erstes freies Ende (26) und ein zweites Ende zur Befestigung (28), der genannte Korpus (22) ist aus einem zweiten Material hergestellt, das härter ist als das erste Material, und einer Basis (24; 24'; 24"), die in deutlich kreisförmiger Form das zweite Ende (28) des Korpus des greifbaren Teils mit einer peripheren Zone (16) des optischen Teils verbindet, wobei die genannte Basis (24; 24'; 24") mit der Peripherie des optischen Teils auf einer ausreichenden Länge verbunden ist, entsprechend einem Winkel B zur Mitte unter 180 Grad und über 45 Grad, um eine Torsionsbewegung des optischen Teils zum greifbaren Teil (20; 20'; 20") zu vermeiden.

2. Intraokulares Implantat nach Anspruch 1, **dadurch gekennzeichnet, daß** es über einen greifbaren Teil (20) verfügt, der aus einem deutlich ringförmigen Korpus (22) besteht, deutlich konzentrisch und komplanar zum optischen Teil, der genannte Korpus (22) verfügt mindestens über eine Öffnung, ein erstes freies Ende (26) und ein zweites Ende zur Befestigung (28). Der genannte Korpus (22) ist aus einem zweiten Material hergestellt, das härter ist als das erste Material, und einer Basis (24), die in deutlich kreisförmiger Form das zweite Ende (28) des Korpus des greifbaren Teils mit einer peripheren Zone (16) des optischen Teils verbindet, die genannte Basis (24) ist aus dem ersten Material hergestellt und stellt einen Seitenbereich (29a) dar, bestehend aus einer harten Umhüllung (30a), hergestellt aus dem zweiten Material, das den genannten Korpus mindestens bis zum Rand (16) des optischen Teils verlängert.

3. Intraokulares Implantat nach Anspruch 2, **dadurch gekennzeichnet, daß** die genannte Basis (24) über einen ersten Seitenbereich (29a) verfügt, bestehend aus einer harten Umhüllung (30a), hergestellt aus dem zweiten Material, das den genannten Korpus mindestens bis zum Rand (16) des optischen Teils verlängert und einem zweiten Seitenbereich (29b), bestehend aus einer harten Umhüllung (30b), hergestellt aus dem zweiten Material, die sicht nicht über die gesamte Länge dieses zweiten Seitenbereichs erstreckt und so an den optischen Teil grenzt, daß mindestens ein Durchmesser (34) des optischen Teils besteht, der sich auf der Basis des greifbaren Teils erstreckt, ohne die genannten Umhüllungen (30a, 30b) zu durchschneiden.

4. Intraokulares Implantat nach Anspruch 3, **dadurch gekennzeichnet, daß** die äußere Kontur der genannten Seitenbereiche (29a, 29b) der Basis (24) des greifbaren Teils gekrümmt ist.

5. Intraokulares Implantat nach einem der Ansprüche 3 oder 4, **dadurch gekennzeichnet, daß** die äußere Kontur der genanten Seitenbereiche (29a, 29b) der Basis (24) des greifbaren Teils so ausgeführt ist, daß sie im Verbindungsbereich zwischen dem peripheren Bereich (16) des optischen Teils und der Basis (24) des greifbaren Teils eine Hohlkehle für den ersten Seitenbereich (29a) und eine Tangentialverlängerung des äußeren Randbereichs des optischen Teils (12) für den zweiten Seitenbereich (29b) bildet.

6. Intraokulares Implantat nach einem der Ansprüche 3 bis 5, **dadurch gekennzeichnet, daß** die harten Umhüllungen (30a, 30b) über eine deutlich konstante Breite verfügen, die der äußeren Form der Seitenbereiche (29a, 29b) folgt.

7. Intraokulares Implantat nach einem der Ansprüche 3 bis 6, **dadurch gekennzeichnet, daß** die harten Umhüllungen (30a, 30b) sich zu den Seitenflächen der Basis (24) hin öffnen.

8. Intraokulares Implantat nach einem der Ansprüche 3 bis 7, **dadurch gekennzeichnet, daß** das zweite Material mindestens einen Teil der Stärke von Teilen von Seitenbereichen (29a, 29b) ausfüllt, darin enthalten auch die Umhüllungen (30a, 30b).

9. Intraokulares Implantat nach einem der vorangegangenen Ansprüche, **dadurch gekennzeichnet, daß** der ringförmige Korpus (22) mindestens einen Kreisbogen des Mittelpunktwinkels von 270° bedeckt.

10. Intraokulares Implantat nach einem der Ansprüche 2 bis 9, **dadurch gekennzeichnet, daß** die harte Umhüllung oder die harten Umhüllungen (30a, 30b) sich bis auf den Randbereich (17) des optischen Teils (12) erstrecken.

11. Intraokulares Implantat nach einem der vorangegangenen Ansprüche, **dadurch gekennzeichnet, daß** es sich bei dem zweiten Material um PMMA handelt.

12. Intraokulares Implantat nach einem der vorangegangenen Ansprüche, **dadurch gekennzeichnet, daß** das erste Material ein hydrophiles Acrylat enthält.

13. Intraokulares Implantat nach einem der vorangegangenen Ansprüche, **dadurch gekennzeichnet, daß** es sich bei dem ersten Material um Silikongel handelt.

14. Intraokulares Implantat nach einem der vorangegangenen Ansprüche, **dadurch gekennzeichnet, daß** die Basis (24") über eine Aushöhlung (36) verfügt, die geeignet ist das Zusammenführen eines vorherigen und späteren Teils von Seitenwänden eines Kapselbeutels eines Auges, in den das genannte Implantat eingebracht werden soll, zu ermöglichen,

## Claims

1. Intraocular implant (10) comprising a substantially circular optical part (12) made of a first flexible material enabling it to be folded along a diameter and a haptic part (20; 20'; 20"), the haptic part (20; 20'; 20") comprising a substantially ring-shaped body (22), substantially concentric and coplanar to the optical part, said body (22) at least presenting an opening, a first free end (26) and a second connecting end (28), said body (22) being made of a second material more rigid than the first material, and a base (24; 24'; 24") connecting in substantially radial manner the second end (28) of the body of the haptic part to a peripheral zone (16) of the optical part, said base (24; 24'; 24") being connected to the periphery of the optical part over a sufficient length corresponding to an angle at the centre b less than 180 degrees and more than 45 degrees in order to avoid a movement of torsion of the optical part with respect to the haptic part (20; 20'; 20").

2. Intraocular implant according to Claim 1, **characterised in that** the haptic part (20) comprises a substantially annular body (22), substantially concentric and coplanar to the optical part, said body (22) at least presenting an opening, a first free end (26) and a second connecting end (28), said body (22) being made of a second material more rigid than the first material, and a base (24) connecting in substantially radial manner the second end (28) of the body of the haptic part to a peripheral zone (16) of the optical part, said base (24) being made with the first material and presenting a lateral zone (29a) comprising a rigid armature (30a) made with the second material which extends said body at least up to the periphery (16) of the optical part.

3. Intraocular implant according to Claim 2, **characterised in that** said base (24) presents a first lateral zone (29a) comprising a first rigid armature (30a) made with the second material which extends said body at least up to the periphery (16) of the optical part and a second lateral zone (29b) comprising a second rigid armature (30b) made with the second material, not extending over the whole length of this second lateral zone and adjacent the optical part so that there exists at least one diameter (34) of the optical part which extends in the base of the haptic part without cutting said armatures (30a, 30b).

4. Intraocular implant according to Claim 3, **characterised in that** the outer contour of said lateral zones (29a, 29b) of the base (24) of the haptic part is curved.

5. Intraocular implant according to Claim 3 or 4, **characterised in that** the outer contour of said lateral zone (29a, 29b) of the base (24) of the haptic part is shaped so as to form, at the level of the zone of join between the peripheral zone (16) of the optical part and the base (24) of the haptic part, a groove for the first lateral zone (29a) and a tangential extension of the outer contour of the optical part (12) for the second lateral zone (29b).

6. Intraocular implant according to any one of Claims 3 to 5, **characterised in that** the rigid armatures (30a, 30b) present a substantially constant width which follows the shape of the outer contour of the lateral zones (29a, 29b).

7. Intraocular implant according to any one of Claims 3 to 6, **characterised in that** the rigid armatures (30a, 30b) open out on the lateral faces of the base (24).

8. Intraocular implant according to any one of Claims 3 to 7, **characterised in that** the second material fills at least a part of the thickness of the parts of the lateral zones (29a, 29b) comprising the armatures (30a, 30b).

9. Intraocular implant according to any one of the preceding Claims, **characterised in that** the annular body (22) covers an arc of circle with angle at the centre at least substantially equal to 270°.

10. Intraocular implant according to any one of Claims 2 to 9, **characterised in that** the rigid armature or armatures (30a, 30b) extend up to the peripheral zone (17) of the optical part (12).

11. Intraocular implant according to any one of the preceding Claims, **characterised in that** the second material is PMMA.

12. Intraocular implant according to any one of the preceding Claims, **characterised in that** the first material includes a hydrophilic acrylate.

13. Intraocular implant according to any one of the preceding Claims, **characterised in that** the first material is silicone gel.

14. Intraocular implant according to any one of the preceding Claims, **characterised in that** the base (24") comprises a recess (36) adapted to allow merging of a portion of the anterior and posterior walls of a capsular sac of an eye in which said implant is intended to be housed.
